# EUROPEAN PATENT SPECIFICATION

(11) **EP 0 406 584 B1**
(45) Date of publication and mention of the grant of the patent: **07.09.1994**
(21) Application number: 90110985.0
(22) Date of filing: 11.06.1990
(51) Int. Cl.: A61M 39/00

(54) **Cap for medical connector**
Kappe für medizinisches Verbindungsstück
Capot de protection pour raccord médical

(30) Priority: 09.06.1989 JP 145225/89
(43) Date of publication of application: 09.01.1991
(73) Proprietor: TERUMO KABUSHIKI KAISHA, Tokyo 151 (JP)
(72) Inventor: Kira, Norisuke, Fujinomiya-shi, Shizuoka-ken (JP)
(74) Representative: Casalonga, Axel

(56) References cited:
- EP-A- 0 260 393
- DE-A- 2 818 146
- DE-U- 8 906 628

## Description

### BACKGROUND OF THE INVENTION

### Field of the Invention:

This invention relates to a cap for use with a medical tool connector as disclosed in claim 1. More particularly, it relates to a cap for use with such a medical tool connector as a connector of the type to be fitted to the leading end of a tube extended as a connector from a dialytic liquid bag which is used as in the method of peritoneal dialysis, for example.

### Description of the Prior Art:

In the mutual connection of tubes or in the connection of a tube to a container or to a catheter involved in preparation for the continuous therapy such as peritoneal dialysis, solution transfusion, or blood transfusion, perfect prevention of the tube interior from ingression of microorganisms poses itself a technical task. Particularly in the peritoneal dialysis, a therapy to be performed at such a site as the abdominal cavity which has absolutely no ability to protect against microorganisms, infallible prevention of the ingression of microorganisms through the tube into the abdominal cavity is regarded as an absolute technical task. The latest therapy by peritoneal dialysis is such that the apparatus and tools to be used therefor are not voluminous and the cost incurred thereby is conspicuously small as compared with the dialysis resorting to an artificial kidney. Since the cause for peritoneal coalescence has been substantially elucidated, the peritoneal dialysis can be performed without inducing peritoneal coalescence. Moreover, the therapy itself has been developed to the extent of appreciably lowering the burden exerted on the patient. Further, the method of continuously applicable peritoneal dialysis (CAPD method) which allows the patient to take an uninterrupted treatment while continuing his work has been invented and employed widely for practical use. In these advantageous circumstances, this therapy has been reawakening interest and attracting attention. The reliability of this method of dialysis with respect to the safety of life hinges on the microorganisms such as bacteria and viruses into the tube can be infallibly prevented and, consequently, the complication of peritonitis due to the propagation of such microorganisms within the abdominal cavity can be avoided. It is held at present that protracted performance of this therapy is not completely feasible.

As a typical example, the conventional method of continuously applicable peritoneal dialysis will be described below. By a surgical operation, a catheter is implanted in a patient's abdominal cavity and a connector member is attached the the extracorporeal end of the implanted catheter or the catheter having the connector member attached in advance thereto is wholly implanted in the patient's abdominal cavity. Then, a connector member attached to the leading end of a tube of a dialytic liquid bag is connected to the connector member of the implanted catheter either directly or through the medium of a tube having connector members attached one each to the opposite ends thereof. The bag is suspended at a prescribed height higher than the abdominal cavity so that the dialytic liquid contained in the bag will flow down into the catheter and induce the dialysis aimed at. In this case, for the purpose of preventing the ingression of microorganisms during the course of the connection of the connector members, the two connector members, for example, are sterilized with flame and connected to each other by shrinkage fit.

The dialytic liquid bag is stowed in a packing container in conjunction with a dialytic liquid discharge tube attached to the bag and provided at the leading end thereof with a connector member and is preserved in a wholly sterilized state. Further, this connector member is designed so as to keep the tube in a thoroughly sterilized state even after the cap is attached thereto and the packing container is opened.

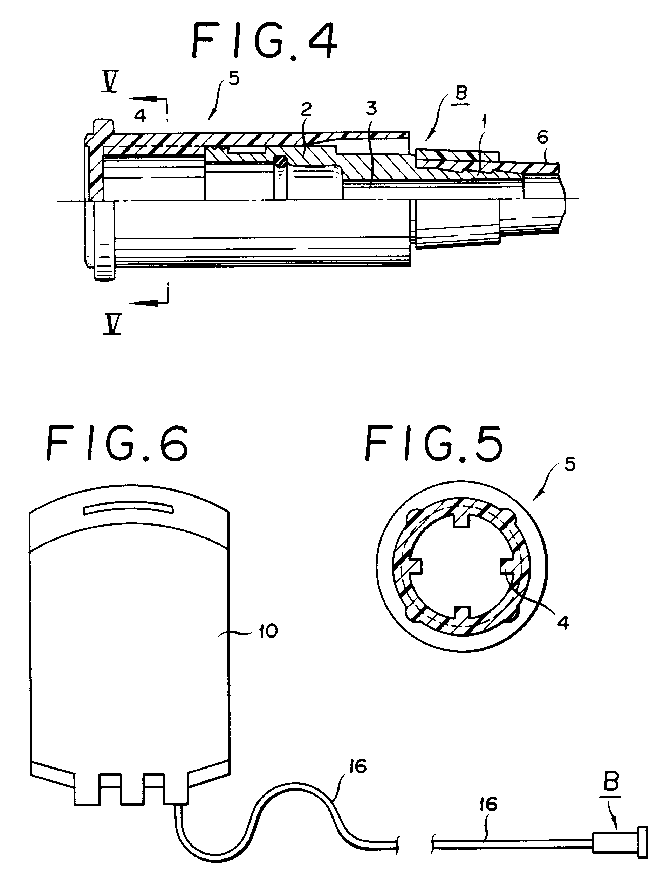

The conventional cap for the connector is made of a heat-resistant corrosionproof metal, as illustrated in Fig. 4 and Fig. 5, in the shape of a blind cylindrical cap 5 provided at one end thereof with a tube insertion terminal part 1 having a substantially equal diameter in the direction of the terminal, at the other end thereof with a female insertion terminal part 2 capable of continuously accommodating therein a male insertion terminal part of another connector member (not shown), and in the interior thereof with a stopper 4 comprising a plurality of ridges 4 formed on the lower inner wall surface so as to be inserted into a female connector member B formed in the shape of a short tube possessing a flow path 3 communicating with the aforementioned other connector member (US-A-4,588,402, 4,668,271, and 4,820,288). A further example of a prior art cap is given in EP-A-0 260 393.)

When the dialytic solution bag has the conventional cap of this description fitted around the connector member B at the leading end of the flexible tube 6 of the bag, it is set in place in a packing container (such as, for example, a bag of flexible vinyl chloride resin), sealed hermetically, and subjected to about 30 minutes' steam sterilization in an autoclave at 120°C. In consequence of this sterilization in the autoclave, the interior of the dialytic liquid bag is sterilized by the fact that the steam permeating into the container passes through the flexible tube wall and finds its way into the interior of the tube 6 and the interior of the flow path 3 of the connector member B.

The cap intended to protect the connector member at the leading end of the flexible tube is inherently desired to be capable of substantially completely sealing the connector member so as to keep the interior of the connector member and the interior of the tube in an aspectic state until immediately before use. When the dialytic liquid bag using the cap of this nature is sterilized in the autoclave, the steam entrapped in the hermetically sealed bag is condensed after completion of the sterilization. This condensation of steam has the possibility of crushing or flattening the flexible tube and even inducing the phenomenon of blocking of the flexible tube. When the sterilization is carried out with ethylene oxide gas, it is difficult to sterilize the interior of the flexible tube perfectly because this gas does not have enough ability to permeate into the flexible tube.

The problems mentioned above have been heretofore precluded by providing the cap with ribs or recesses adapted to permit passage of air or forming grooves in the connector member. Logically, these measures may well be regarded as disadvantages from the standpoint of maintenance of the aspectic state mentioned above.

Moreover, since this cap is generally made of such thermoplastic resin as polypropylene and, therefore, is thermally shrunken under the influence of the autoclave sterilization, it must be molded in a slightly larger size enough to make up for this shrinkage. It has occurred at times that the cap will slip off the connector member between the time the cap is attached to the connector member and the time the dialytic liquid bag placed in the autoclave undergoes the sterilization.

An object of this invention, therefore, is to provide a novel cap for the connector used in the medical tool.

Another object of this invention is to provide a cap for the connector in the medical tool, which cap has only a sparing possibility of slipping off the connector member during the course of sterilization.

### SUMMARY OF THE INVENTION

The objects described above are accomplished by a cap for the connector in the medical tool, which cap comprises a blind cylindrical body provided on the bottom thereof with means of closing an open end of a connector member desired to be capped and an air permeable filter member disposed on said closing means.

The present invention also discloses a cap for the connector in the medical tool, wherein the closing means is so constructed as to be projected from the bottom of the cylindrical body toward the open end thereof in the shape of a tube coaxially relative to the cylindrical body and to be inserted into the open end of the connector member. The present invention further discloses a cap for the connector in the medical tool, wherein the filter member is disposed so as to close an open part formed in the bottom part of the tubularly projected part. The present invention further discloses a cap for the connector in the medical tool, wherein the tubularly projected part is formed integrally with the cylindrical body. The present invention further discloses a cap for the connector in the medical tool, wherein the tubularly projected part is made of thermoplastic resin. The present invention further discloses a cap for the connector in the medical tool, wherein the outer leading end part of the tubularly projected part is formed in a converging shape.

The present invention further discloses a medical tool, characterized by the fact that the medical tool is provided with a tube having one end thereof closed and the other end thereof fitted with a connector member and a cap for the connector in the medical tool constructed as described above is fitted around the open end of the connector member. The present invention is directed to a cap for the connector in the medical tool, which cap comprises a blind cylindrical body provided on the bottom part thereof with means of closing an open end of a connector member desired to be capped and a filter member disposed on the closing means. Owing to this construction, even when the medical tool is sterilized with high-pressure steam in an autoclave and the steam entrapped inside the tool is condensed after completion of the sterilization, the medical tool has no possibility of being crushed because air flows into the tube through the filter member. When the medical tool is left standing between the time the packing container is opened prior to the use of the medical tool and the time the cap is removed from the medical tool or when the tube is pressed or bent so as to inhale or exhale air, the possibility of the tube interior being contaminated with microorganisms is nil because the filter member filtrates the air.

When the closing means is constructed so as to be projected from the bottom part of the cylindrical body to the open end thereof in the shape of a tube coaxially relative to the cylindrical body and to be inserted into the open end of the connector member, since the tubularly projected part can be molded in a size suitable for tight fit, it preferably fits the connector member after the sterilization owing to the shrinkage of the cap. Thus, the present invention has given a solution to the problem that the cap of the conventional construction molded in a slightly large size for insertion around the connector member and allowed to shrink fit it under the thermal influence of sterilization is liable to come off the connector member prior to the sterilization.

### BRIEF DESCRIPTION OF THE DRAWINGS

Fig. 1 is a half-sectioned side view of a cap for a connector in a medical tool according with the present invention,
Fig. 2 is a half-sectioned side view of the cap in the state having a connector member inserted therein,
Fig. 3 is a cross section illustrating the state in which two connector members are joined to each other,
Fig. 4 is a cross section illustrating the state in which the conventional cap is fitted around a connector member,
Fig. 5 is a cross section taken through Fig. 4 along the line V-V, and
Fig. 6 is a diagram typically illustrating a medical tool possessing a connector around which the cap of the present invention is to be fitted.

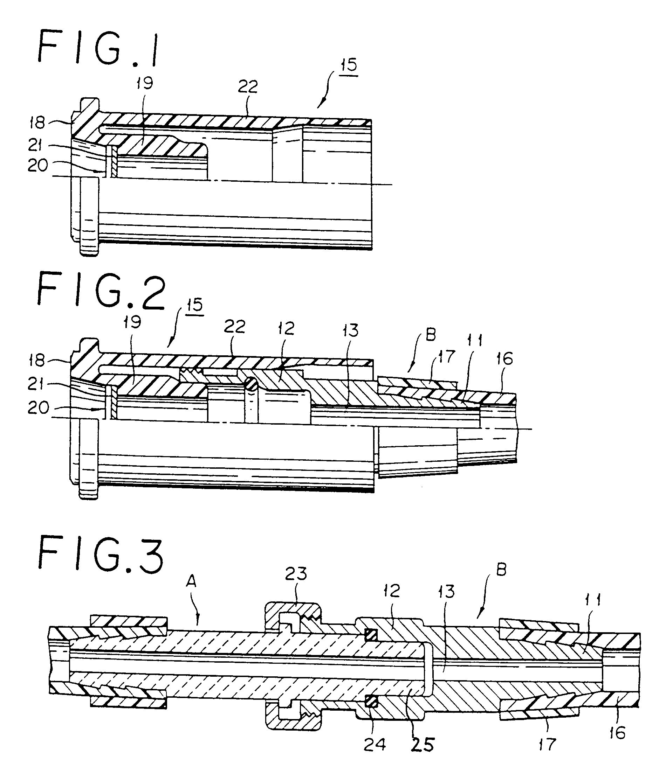

Now, one embodiment of the present invention applied to a connector member at the leading end of a tube communicating with a dialytic solution bag will be described below with reference to the accompanying drawing. A female connector member B is connected through the medium of a tube insertion terminal part 11 as illustrated in Fig. 2 to a dialytic liquid passing tube 16 having one end thereof connected to a dialytic liquid bag 10 as illustrated in Fig. 6, retained in place with an annular retaining member 17, and provided in the interior thereof with a flow path 13. An open end of an inserted part 12 of the female connector member B is closed with closing means which is formed in a cap proper. The closing means, for example, is a tubularly projected part 19 which, as illustrated in Fig. 1 and Fig. 2, is projected from the bottom part of a cylindrical body 15 as a main body of the cap coaxially relative to the cylidrical body 15. An air permeable filter member 21 is attached fast to an open part 20 formed in a bottom part 18 of the tubularly projected part 19.

The connector member B is inserted between an outer barrel 22 of the cylindrical body 15 and the tubularly projected part 19 formed inside the outer barrel 15. An open end of the connector member B is inserted tightly around the tubularly projected part 19. On the other hand, it is inserted rather loosely in the outer barrels 22 in consideration of the shrinkage expected to occur under the thermal influence of sterilization in an autoclave. Since the open end of the connector member B is tightly inserted around the tubularly projected 19 as described above, the connector part B is preferable to have the outer leading end part thereof formed in a converged shape.

The cap, as a whole, is preferable from the standpoint of airtightness of contact with the connector member B and price to be made of thermoplastic resin such as polypropylene, polyethylene, vinyl chloride resin, or polystyrene, for example. The outer barrel 22 and the tubularly projected part 19 are preferable to be integrally formed. The air permeable filter member 21 is formed of filter paper or nonwoven fabric, for example, and is fastened by adhesion, fusion, or other means to the open part 20 formed in the bottom part.

The dialytic solution bag (not shown) having the connector member B thereof covered with the cap of this invention as illustrated in Fig. 2 is stowed in a packing container (not shown), hermetically sealed therein, and then sterilized in an autoclave at temperature of 120°C, for example, for about 30 minutes. At this time, the steam in the autoclave permeates the wall of the packing container (bag of flexible vinyl chloride resin, for example) and enters the container interior and, at the same time, permeates through the wall of the flexible tube (tube of flexible vinyl chloride resin, for example) and enters the tube interior and also finds its way into the passage of the connector member, to effect sterilization of the entire container. After the autoclave sterilization is completed, the partial pressure of steam inside the tube is greatly lowered because the steam inside the tube is condensed. Since the air inside the packing container passes through the filter member add flows into the tube interior, however, the possibility of the tube being crushed or blocked is perfectly nil.

The method of continuously applicable peritoneal dialysis (CAPD method) by the use of the dialytic solution bag is effected by opening the packing container, removing the cap, subjecting the connector member A connected to the catheter retained inside the peritoneum and the connector member B stripped of the cap to flame sterilization with alcohol lamp, then inserting an insertion terminal part 25 of a male connector member A into the female connector member B, locking the two connector members with a locking member 23, and sealing the locked joint watertightly with an O-ring 24.

The connector members A and B are both preferable to be made of a heat-resistant corrosionproof material because they are subjected to the flame sterilization prior to use. The materials which fulfil the requirement include metals, ceramics, and heat-resistant resins, for example. Since these connector members are joined by shrink fit of one sort, it is desirable to have one of them made of a metallic material and the other member made of a ceramic material. It is particularly desirable to have the female connector member made of a metallic material and the male connector member made of a ceramic material. The metallic materials which are usable herein include stainless steel, titanium alloy, nickel, nickel alloy, and chromium-plated brass, for example. The ceramic materials which are usable herein include zirconia, silicon nitride, alumina, steatite, forsterite, silicon carbide, and silica, for example.

### Example

An open end of a female connector member B made of nickel alloy, attached to the leading end of a tube of flexible vinyl chloride resin of a CAPD grade dialytic solution bag, and fixed in place with an annular retaining member 17 as illustrated in Fig. 1 and Fig. 2 was inserted around a tubularly projected part 19 of a cap provided with closing means projected from the bottom part of a cylindrical body of the cap coaxially relative to the cylindrical body and a filter member 21 disposed in an opening 20 formed in the bottom part. The dialytic liquid bag provided with the connector member B covered with the cap as described above was stowed and hermetically sealed in a bag obtained by superposing two sheets each of a three-layer construction of polypropylene-nylon-polypropylene and fusing the edges of the superposed sheets and subjected to autoclave sterilization at 120°C for about 30 minutes. In the test runs using the cap of the present invention, absolutely no separation of cap occurred during the course of autoclave sterilization.

## Claims

1. A cap for a connector in a medical tool, comprising
- a blind cylindrical body (15) having an outer barrel (22),
- means (19) provided on the bottom part of the cylindrical body (15) for closing an open end of a connector member (B) to be capped, wherein the closing means (19) is constructed so as to be tubularly projecting from the bottom part of the cylindrical body (15) toward the open end thereof coaxially relative to the cylindrical body and inserted inside an open end of the connector member (B), wherein the open end of the connector member (B) is inserted between the barrel (22) and the tubular body of the closing member (19) and is fitted tightly around the tubularly projecting closing means (19),
- an air permeable filter member (21) disposed in the closing means (19) for closing an open part formed in the bottom part of thereof,
**characterised** in that
- the tubular body of the closing member (19) is cylindrical and
- the barrel (22) of the cap is made in such a size with respect to the external diameter of the open end of the connector member (B) in order to enable the barrel (22) to shrink fit around the connector member (B) under the thermal influence of sterilization in an autoclave.

2. A cap according to claim 1, wherein the tubularly projected part (19) is formed integrally with the cylindrical body.

3. A cap according to claim 1 or 2, wherein the tubularly projected part (19) is made of thermoplastic resin.

4. A cap according to any of claims 1 to 3, wherein the tubularly projected part (19) has the outer leading end part thereof formed in a converged shape.

5. A medical tool possessing a tube closed at one end thereof and provided at the other end thereof with a connector member (B) and having an open end of the connector member covered with a cap for a connector in a medical tool as set forth in any of claims 1 to 4.

## Patentansprüche

1. Kappe für ein Verbindungsstück in einem medizinischen Gerät, umfassend
- einen zylindrischen Einsatzkörper (15), der ein Außenrohrstück (22) aufweist,
- eine auf dem Bodenteil des zylindrischen Körpers (15) vorgesehene Einrichtung (19) zum Verschließen eines offenen Endes eines Anschlußgliedes (B), das mit einer Kappe versehen werden soll, wobei die Verschlußeinrichtung (19) so aufgebaut ist, daß sie rohrförmig vom Bodenteil des zylindrischen Körpers (15) zu dessen offenem Ende hin koaxial in bezug auf den zylindrischen Körper vorsteht und innenseitig eines offenen Endes eines Anschlußgliedes (B) eingesetzt wird, wobei das offene Ende des Anschlußgliedes (B) fest um die rohrförmig vorstehende Verschlußeinrichtung (19) angebracht ist,
- ein luftpermeables Filterelement (21), das in der Verschlußeinrichtung (19) zum Verschließen eines in dessen Bodenteil gebildeten offenen Teils vorgesehen ist, wobei das offene Ende des Anschlußgliedes (B) zwischen dem Rohrstück (22) und dem rohrförmigen Körper des Verschlußelementes (15) fest um den rohrförmigen Körper herum eingesetzt ist,
dadurch **gekennzeichnet,** daß
- der rohrförmige Körper des Verschlußelementes (19) zylindrisch ist und das Rohrstück (22) der Kappe in einer etwas größeren Form in bezug auf den Außendurchmesser des offenen Endes des Anschlußgliedes (B) geformt ist, um das Rohrstück (22) in die Lage zu versetzen, unter der Wärmeeinwirkung der Sterilisation in einem Autoklaven einen Schrumpfsitz um das Anschlußglied B zu bilden.

2. Kappe nach Anspruch 1, bei der der rohrförmig vorstehende Teil (19) integral mit dem zylindrischen Körper gebildet wird.

3. Kappe nach Anspruch 1 oder 2, bei der der rohrförmig vorstehende Teil (19) aus thermoplastischem Harz hergestellt ist.

4. Kappe nach einem beliebigen der Ansprüche 1 bis 3, bei der der äußere Vorderendteil des rohrförmig vorstehenden Teils (19) in einer konvergierenden Form gebildet ist.

5. Medizinisches Gerät, aufweisend einen Schlauch, der an einem Ende verschlossen ist und am anderen Ende mit einem Anschlußglied (B) versehen ist und bei dem ein offenes Ende des Anschlußgliedes mit einer Kappe für ein Anschlußstück in einem medizinischen Gerät überdeckt ist, wie in einem beliebigen der Ansprüche 1 bis 4 dargestellt worden ist.

## Revendications

1. Coiffe de protection pour un raccord d'outil médical qui comprend :
- un corps cylindrique aveugle (15) présentant une douille extérieure (22),
- un moyen (19) placé sur la partie de base du corps cylindrique (15) pour fermer une extrémité ouverte d'un élément de raccord (B) qui doit être protégé avec la coiffe, dans lequel le moyen de fermeture (19) est construit pour être une projection tubulaire de la partie de base du corps cylindrique (15) en direction de l'extrémité ouverte de celui-ci, de façon coaxiale par rapport au corps cylindrique, et pour être inséré dans l'extrémité ouverte de l'élément de raccord (B), sachant que l'extrémité ouverte de l'élément de raccord (B) est introduite entre la douille (22) et le corps tubulaire du moyen de fermeture (19) et étroitement emboîtée autour du moyen de fermeture (19) tubulaire qui dépasse,
- un élément formant filtre (21), perméable à l'air, placé dans le moyen de fermeture (19) pour fermer une partie ouverte formée dans la partie de base de celui-ci,
**caractérisée** en ce que :
- le corps tubulaire de l'élément de fermeture (19) est cylindrique, et
- la douille (22) de la coiffe est réalisée avec une taille telle par rapport au diamètre extérieur de l'extrémité ouverte de l'élément de raccord (B) qu'elle permet à la douille (22) de s'emboîter par rétrécissement autour de l'élément de raccord (B) sous l'effet thermique d'une stérilisation en autoclave.

2. Coiffe de protection selon la revendication 1, dans laquelle la partie tubulaire qui dépasse (19) est formée en un seul bloc avec le corps cylindrique.

3. Coiffe de protection selon la revendication 1 ou 2, dans laquelle la partie tubulaire qui dépasse (19) est faite d'une résine thermoplastique.

4. Coiffe de protection selon l'une quelconque des revendications 1 à 3, dans laquelle la partie tubulaire qui dépasse (19) a sa partie d'extrémité avant extérieure qui a une forme convergente.

5. Outil médical possédant un tube fermé en une extrémité et muni en son autre extrémité d'un élément de raccord (B) et ayant une extrémité ouverte de l'élément de raccord qui est couverte par une coiffe de protection destinée à un raccord d'outil médical tel que défini dans l'une des revendication 1 à 4.
